# EUROPEAN PATENT APPLICATION

(11) **EP 4 368 114 A1**
(43) Date of publication of application: **15.05.2024**
(21) Application number: 23208668.6
(22) Date of filing: 08.11.2023
(51) Int. Cl.: A61B 8/00, A61B 8/08, G06T 5/00, G06T 7/00, G16H 30/20, G16H 30/40

(54) **SYSTEMS AND METHODS FOR TRANSFORMING ULTRASOUND IMAGES**

(30) Priority: 08.11.2022 US 202263423777 P
(71) Applicant: BFLY Operations, Inc., Burlington, MA 01803 (US)
(72) Inventor: Samangouei, Pouya, San Francisco CA, 94102 (US); Omar, Murad, Seacacus NJ, 07054 (US); Cohen, Joseph, Portland OR, 97230 (US); Brattain, Eric, New Paltz NY, 12561 (US); lovchinsky, Igor, New York NY, 12561 (US); Silberman, Nathan, Brooklyn NY, 11201 (US); Sankaranarayanan, Swami, Hoboken NJ, 07030 (US); Liu, Yang, Hoboken NJ, 07030 (US); Howell, Audrey, Guilford CT, 06437 (US)
(74) Representative: Kelly, Edward

(57) **Abstract**

The systems and methods, in one embodiment, include a convolutional neural network (CNN) model trained by a modified version of the CycleGAN process. The CNN filters ultrasound images generated by a handheld ultrasound device to generate images that are perceptually similar to images generated by a cart-based ultrasound device in terms of quality. The resulting images look sharper and less noisy compared to the original inputs. The invention is a tool within the actions menu of a mobile app. When the tool is open, users can turn filtering on and off. The users will turn filtering on to reduce noise so they can reach the right scanning spot faster. After which because of body habitus, still there might be some noise that this tool can clean up. The user can then decide to have the filtering on or off during the diagnostic process.

## Description

### FIELD

The systems and methods described herein relate to ultrasound imaging for medical imaging applications and to user interfaces that adjust image characteristics to make clinical conduct of imaging procedures more facile.

### BACKGROUND

Scientists and engineers have made remarkable advances with medical imaging technologies, including ultrasound imaging probes that are portable, provide ease of use and ready transport and produce clinically excellent images. Such devices allow doctors and other clinicians to use medical imaging for patients who are remote from hospitals and clinics with imaging equipment or who treat patients who are confined to their homes. As such, these portable systems provide patients access to the excellent care that advanced medical imaging can provide.

Examples of such portable imaging systems include those described in US Patent 10,856,840 which is granted to the assignee hereof. These new handheld ultrasound imaging devices have demonstrated accuracy similar to that provided by cart-based ultrasound machines. See Le et al.; Comparison of four handheldpoint-of-care ultrasound devices by expert users; The Ultrasound Journal (2022) 14:27. In studies, such as the study set out in the above cited publication of Le et al., multiple ultrasound imaging applications were considered, including bedside procedures, such as thoracentesis and epidural analgesia, and diagnostic evaluation of left ventricular function. Handheld ultrasound imaging devices performed well and in these studies and were found to be as clinically effective as cart-based ultrasound systems.

Although handheld ultrasound imaging devices have been shown in studies to be as clinically effective as cart-based ultrasound imaging systems, there are differences between the cart-based devices and the handheld devices. Some of these differences appear in how images are rendered. Cart-based ultrasound devices may generate images with a sharper and cleaner look than handheld systems. These differences in appearance may arise from the different device form factors and the differences in components used in the two different systems. The larger form factor of cart-based systems allows for the use of different components from those used in the smaller handheld units. This can include components, such as power supplies and transmitters, that are larger, more power-hungry, more expensive, and higher performance, but are less suited for use in a battery powered handheld device. Collectively, these different components can cause the appearance of images generated by cart-based systems to look different from images generated by handheld ultrasound systems.

As used herein, a handheld ultrasound imaging device encompasses, but is not limited to, ultrasound devices that are free from the cart carried power and processing systems used by cart-based ultrasound devices. They typically include a handheld probe and a mobile phone or table that connects to the probe. Typically, they are battery powered and the battery is carried in the probe. In any case, the handheld device typically, but not always, are physical devices which can generate and receive ultrasonic pulses and that have a size, weight, and power requirements such that it can be utilized by hand and without the need to be attached to an external source of power or processing units to generate clinically useful ultrasound images. Of course, carts can be associated with these handheld devices, but the handheld device does not rely on the cart for power and processing during operations. It will be apparent to those skilled in the art that in alternate embodiments handheld ultrasound imaging devices can encompass other elements based on the application at hand.

Additionally, cart-based devices typically have larger image display screens as well as different image display resolutions. Resolution as it is used in this embodiment includes, but is not limited to, a visual characteristic of an image which defines the amount of detail which an image holds such that an image which holds more detail is categorized as having a higher level of resolution. It will be clear to those skilled in the art that in other embodiments this term can encompass other elements based on the application at hand. Further, some cart-based devices have specialized graphics processors for more sophisticated image processing. As such, the clinician can experience a different visual aesthetic for the images produced by a cart-based ultrasound device as compared to a handheld ultrasound device. Moreover, the images generated by cart-based systems may have sharper features and a cleaner, less cluttered appearance. This may help clinicians find the anatomical structure of interest, such as a heart chamber, more quickly and allow the clinician to make a more accurate and faster diagnosis. Further, some clinicians may have a comfort with the more familiar visual aesthetic provided by cart-based ultrasound devices.

Although these portable ultrasound systems work well, are clinically effective and offer significant advantages over cart-based ultrasound devices, there remains a need to improve the ease at which clinicians who are facile with operating cart-based devices, can transition to use of handheld ultrasound devices and as such there is a remaining need for improved systems.

### SUMMARY

The systems and methods described herein provide, in one aspect, a selectable image filter that can adjust an image collected by the handheld ultrasound device and transform that image using a neural filter that renders the image with the look and feel of a cart-based ultrasound device. In one embodiment, the clinician-user can activate this filter while seeking the proper position for the ultrasound probe on the habitus of the patient. The clinician-user may toggle the filter between an active and inactive state during the positioning and imaging in order to employ adjusted images during the probe location and orientation process and to employ raw, that is unadjusted, images during study of a target anatomical feature.

Further disclosed herein are systems for generating ultrasound images during a medical imaging procedure, comprising an ultrasound imaging device for generating a stream of image data, an image processor for processing the stream of image data to generate images for use by a clinician, a user interface for controlling operation of the image processor and having a neural filter UI switch that activates a neural filter, and a neural filter responsive to the UI switch for processing image data within the stream of images by adjusting an output distribution of the image data to conform with an output distribution of image data generated by cart-based ultrasound images and to maintain content of the image data within the generated stream of image data. Optionally, the system may have the UI switch associated with a cardiac preset configuration for generating cardiac image data. Further optionally, adjusting an output distribution may include adjusting an output distribution of an image to match an image output distribution associated with a cart-based ultrasound imaging device. Still further optionally, the neural filter may include a mapping function for translating image data generated from a handheld ultrasound device to images of the type generated by cart-based ultrasound devices. The neural filter may process paired image data to generate the mapping function or may process unpaired image data to generate the mapping function. Further optionally, the system may include a training module that has and employs a cycle-consistent adversarial network to process unpaired image data to generate the mapping function. One advantage of the systems and methods described herein is that such systems and methods generate the level of ultrasound imaging quality produced by the high-power processors of cart-based ultrasound systems, often having specialized and power intensive image processing circuits, using the relatively low power methods of a neural filter. Still other embodiments may be realized and will be apparent from the figures and disclosure below.

In particular, in certain embodiments the systems and methods described herein include systems of generating ultrasound images during a medical imaging procedure comprising, a handheld ultrasound imaging device for generating a stream of image data from the habitus of the patient, and an image processor for processing the stream of image data to produce images. Additionally, there may be a neural filter which receives the stream of image data from the handheld ultrasound imaging device and processes it to generate a new stream of image data that produces images such that the output distribution of images conforms to the visual properties of the image distribution of ultrasound images of the type produced by cart-based ultrasound systems, and a user interface control for controlling operation of the neural filter and having a UI switch for activating the neural filter. Optionally, the UI switch may comprise a preset configuration for configuring the handheld ultrasound imaging device to generate image data for an associated image study requirement associated with the preset and for processing generated image data with the neural filter to conform the visual properties of the generated image data to have an image distribution of ultrasound images of the type produced by cart-based ultrasound systems for the respective preset image study requirements. Optionally, adjusting the output distribution to conform to the visual properties of the image distribution of the type produced by cart-based ultrasound systems may include adjusting the image data such that the measures of the visual properties of sharpness, resolution, and noise of the resulting output image conform to the measures of the visual properties sharpness, resolution and noise of the output distribution of ultrasound images produced by cart-based ultrasound imaging systems. Further optionally, the neural filter may include a mapping function for translating image data produced by a handheld ultrasound imaging system into image data of the type produced by cart-based ultrasound imaging systems by employing a training module to define for the neural filter visual properties of image data produced by handheld ultrasound imaging devices and cart-based ultrasound imaging systems respectively. Further optionally, the training module may process the image data of paired images across an image distribution of the type produced by handheld ultrasound imaging systems and an image distribution of the type produced by cart-based ultrasound imaging systems to generate the mapping function. Further optionally, the training module may process the image data of unpaired images across an image distribution of the type produced by handheld ultrasound imaging systems and an image distribution of the type produced by cart-based ultrasound imaging systems to generate the mapping function. Optionally the training module may employ a cycle-consistent adversarial network to evaluate unpaired images translated from a first image distribution into a second image distribution to determine the accuracy of the translation, and evaluating images translated from a first image distribution into a second image distribution and then back into the first image distribution to determine the content lost across the translation to generate the mapping function.

In another aspect the methods described herein include a method of generating ultrasound images during a medical imaging procedure comprising, generating with a handheld ultrasound imaging device a stream of image data from the habitus of the patient, and processing the stream of image data to produce images, and receiving the stream of image data and processing it to generate a new stream of image data that produces images such that the output distribution of images conforms to the visual properties of the image distribution of ultrasound images of the type produced by cart-based ultrasound systems, and controlling operation of the neural filter comprising a UI switch for activating the neural filter. Additionally there may be a method for controlling operation of the neural filter including accessing a preset configuration for configuring the handheld ultrasound imaging device to generate image data for image study requirements associated with the preset and processing generated image data with the neural filter to conform the visual properties of the generated image data to have an image distribution of ultrasound images of the type produced by cart-based ultrasound systems for the respective preset image study requirements . Optionally, adjusting the output distribution to conform to the visual properties of the image distribution of the type produced by cart-based ultrasound systems may include adjusting the image data such that the measures of the visual properties of sharpness, resolution, and noise of the resulting output image conforms to the measures of the visual properties of sharpness, resolution and noise of the output distribution of ultrasound images produced by cart-based ultrasound imaging systems. Optionally receiving the stream of image data and processing it to generate a new stream of image data may include employing a neural filter which includes employing a mapping function for translating image data produced by a handheld ultrasound imaging system into image data of the type produced by cart-based ultrasound imaging systems by employing a training module to define for the neural filter visual properties of image data produced by handheld ultrasound imaging devices and cart-based ultrasound imaging systems respectively. Optionally employing a training module may include processing the image data of paired images across an image distribution of the type produced by handheld ultrasound imaging systems and an image distribution of the type produced by cart-based ultrasound imaging systems to generate the mapping function. Further optionally employing a training module may include processing the image data of unpaired images across an image distribution of the type produced by handheld ultrasound imaging systems and an image distribution of the type produced by cart-based ultrasound imaging systems to generate the mapping function. Further optionally employing a training module may include employing a cycle-consistent adversarial network to evaluate unpaired images translated from a first image distribution into a second image distribution to determine the accuracy of the translation, and evaluating images translated from a first image distribution into a second image distribution and then back into the first image distribution to determine the content lost across the translation to generate the mapping function.

### BRIEF DESCRIPTION OF THE DRAWINGS

The systems and methods described herein are set forth in the appended claims. However, for purpose of explanation of these systems and methods, several embodiments are set forth in the following figures and the related description.
FIG. 1 depicts one embodiment of the systems described herein;
FIG. 2 depicts pictorially an application executing on the handheld device of FIG. 1 for adjusting images to translate images from a handheld device domain to a cart-based device domain;
FIG. 3 depicts pictorially a system for training a neural filter of the type employed by the system of FIG. 2;
FIG. 4 depicts a process for controlling an adversarial loss;
FIG. 5 depicts a process for controlling a cyclical consistency loss;
FIG. 6 depicts a first image rendered as a side-by-side presentation of a ground image on the left and an opposing translated image on the right generated by application of a neural filter of the type described with reference to FIG. 3;
FIG. 7 depicts a second image rendered in a side-by-side presentation with the ground image on the left and the translated image on the right having been generated by use of a neural filter of the type described with reference to FIG. 3;
FIG. 8 is a flow chart of one embodiment of the methods described herein;
FIG. 9 depicts a flow chart of another embodiment of the methods described herein; and
FIG. 10 depicts pictorially a clinician applying a neural filter as described herein during locating of the probe of a handheld ultrasound imaging system.

### DETAILED DESCRIPTION

In the following description, numerous details are set forth for purpose of explanation. However, one of ordinary skill in the art will realize that the embodiments described herein may be practiced without the use of these specific details. Further, for clarity, well-known structures and devices are shown in block diagram form to not obscure the description with unnecessary detail.

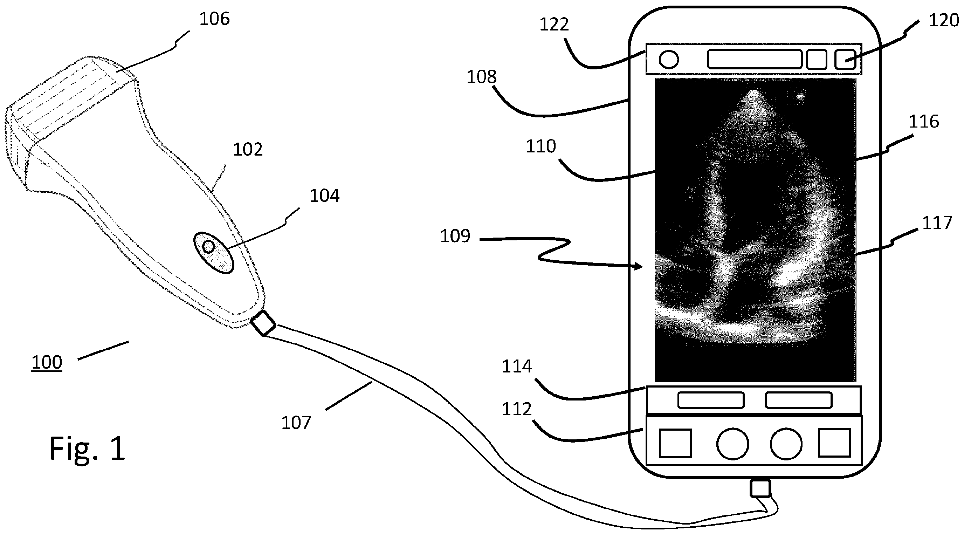

In one embodiment, the systems and methods described herein include, among other things, the system 100 depicted in FIG. 1. FIG. 1 depicts that system 100 includes a probe 102 and a handheld device 108. The probe 102 has a UI control button 104 and a transducer head 106. The probe 102 is coupled by a cable 107 to a handheld device 108, depicted in FIG. 1 as a mobile phone. The depicted handheld device 108 is executing an application 109 that collects data, including image data, from the probe 102. The application 109 may display an image within the image window 110. The image displayed in image 110 may be an anatomical image generated by a clinician applying the probe 102 to the habitus of a patient. Additionally, FIG. 1 depicts that the handheld device 108 may include software UI windows, such as the depicted software UI window 112, which contains one or more software control icons for adjusting the operation of the probe 102.

The probe 102, in this embodiment, is an ultrasound probe of the type disclosed in US Patent 10,856,840, assigned to the assignee hereof. The probe 102 is a handheld ultrasonic imaging probe that can be used by the clinician to image a patient and collect medical images useful in the clinical process of diagnosing and treating the patient. In the depicted embodiment the probe 102 is a handheld battery powered unit, although in other embodiments the probe 102 may draw power from the handheld device 108 or from a remote power supply. The probe 102 has a transducer head 106 that the clinician may place against the tissue of the patient, such as by placing the transducer head 106 in contact with the patient's chest proximate to the heart of the patient or proximate the carotid artery. The depicted probe 102 has a single UI control button 104, although in other embodiments there may be more than one UI control button, or no UI control button. The depicted probe 102 is an example of an ultrasound imaging device for generating a stream of image data. The stream of image data is a stream of image data generated by ultrasound imaging during a medical imaging procedure and that stream includes reflected ultrasound energy detected by the probe 102 transducer head 106 and capable of being processed to reveal as images the anatomical structures, including organs, bones and tissue, of a patient. The stream of image data herein includes, but is not limited to, a sequence of data being received by a processing unit which when processed produces a visual image represented by the data. This may refer to the sequence of data which the ultrasound probe 102 produces after processing the ultrasound signals it receives, or additionally it may refer to the sequence of data that the neural filter 224 produces after translating an image into another style. It will be clear to those skilled in the art that in alternate embodiments this may encompass other elements depending on the application at hand.

In typical operation, the clinician uses the probe 102 and the application 109 executing on the handheld device 108 to capture and display images of anatomical features of the patient. To this end, the application 109 may render the captured image in the image window 110 for the clinician to view. The UI window 112 may provide the clinician with a series of optional user interface controls that the clinician may use to operate the application 109 executing on the handheld device 108 to change how the captured image is rendered, store the image, mark the image, and perform other types of operations useful during the tomographic procedure.

During a tomographic procedure, the clinician adjusts the position and angle of the probe 102 until an image of interest appears in the image window 110. In some embodiments, the clinician may operate the application program 109 by activating UI controls in window 112 to capture images to study, or activate various functions such as, but not limited to, selecting preset configurations, performing imaging control such as for controlling depth, controlling gain, switching modes, turning color on and/or off, controlling a wireless pairing process, or for soft resetting of the probe. In any case, the application 109 allows the clinician to adjust how images are presented and stored.

In the embodiment depicted in FIG. 1, the handheld device 108 is a programmable device that runs the application 109 that, for example, performs the image display functions, user interface functions, such as allowing the clinician to select presets and capture images from the image stream, and configures the system 100 with any selected preset parameters. In this embodiment, the handheld device 108 may be a smart phone, a tablet, or any other suitable handheld device capable of running application programs and of supporting a data connection to the probe 102. In the depicted embodiment the handheld device 108 couples to the probe 102 by way of the cable 107. However, in alternative embodiments, the handheld device 108 and the probe 102 may have a wireless connection of the type suitable for transferring data and control signals between the two devices. In one example, the wireless connection may be the Bluetooth protocol (IEEE 802.15.1), ultra-wideband (UWB, over IEEE 802.15.3), ZigBee (over IEEE 802.15.4), or Wi-Fi (over IEEE 802.11) connection or a connection using some other protocol for short-range wireless communications, preferably with low power consumption. However, any suitable wireless technology may be used including those that work with narrow bands, employ optical communication, or which use some other suitable technique for exchanging information between the probe 102 and the handheld device 108.

In the embodiment depicted in FIG. 1 the transducer head 106 includes an array of ultrasonic transducer elements. The array of ultrasonic transducers may be an array of MEMs transducer devices, such as an array of capacitive micro-machined ultrasonic transducers (CMUTs) or an array of piezoelectric micromechanical ultrasonic transducers (PMUTs), that are capable of generating ultrasonic waves, including beamformed ultrasonic waves and detecting ultrasonic waves as they return from the patient. In one embodiment, the depicted transducer head 106 includes thousands of transducer elements that operate in coordinated action to create the ultrasound beam used for the image collection. In one example, the transducer head 106 includes a two-dimensional array of thousands of transducer elements formed on a semiconductor die or chip. The die or chip may, in certain embodiments, further contain on-chip processing circuitry including more than one thousand analog-to-digital converters and amplifiers. Embodiments of transducers formed on a semiconductor die or chip are shown in more detail in US Patent 9,067,779 and in US application US2019/0275561. Embodiments of on-chip processing circuitry are shown in more detail in US Patent 9,521,991. In other embodiments, the transducer head may use PMUTs and the A/D converters and amplifiers may be on separate chips or dies and the chips and dies may be mounted on a circuit board or boards. In operation, the transducer head 106 detects ultrasonic waves returning from the patient and these waves may be processed by processing circuitry formed on the same chip as the transducers, a signal processor, a CPU, an FPGA, or any suitable type of processing device, or any combination thereof, which may process the returned ultrasound waves to construct image data. That image data may be used by the application 109 running on the handheld device 108 to create images for the clinician.

In the depicted embodiment, the executing application 109 may display the constructed images, including video images, such as the ultrasound images 116 and 117, in the image window 110 so that the clinician can see live images of the patient as those images are being generated by the probe 102. In operation, the application 109 performs image processing for processing the stream of image data generated by the probe 102 for use by a clinician. Image processing may include analyzing and operating on the stream of image data generated by the probe 102, which when generated by the probe 102 is typically in the form of the energies and frequencies reflected back to the transducer head 106 by the anatomical structure of the patient. The application 109 will process this stream of ultrasound image data to generate frames of video data of the type that can be displayed on a screen and understood and put to use by a clinician.

In the depicted embodiment, the application 109 also provides a UI window 112 that has a series of software control buttons, sometimes called widgets, that the clinician may use for controlling the operation of the probe 102. These controls allow the clinician to change how images, such as image 116 and image 117, are rendered, captured, and displayed on the handheld device 108. In the embodiment of FIG. 1, the application 109 further has a menu 114 that depicts a preset selected by the clinician and an indication of the view selected for the clinician. In the depicted example the application 109 is rendering and displaying within the image window 110 the video images 116 and 117 taken during a cardiac imaging procedure. In this example, the system 100 is configured with a cardiac imaging preset. This cardiac imaging preset configures the system 100 with operating parameters selected for capturing clinically useful images of a procedure that involves imaging a feature of the patient's heart, such as volume of a ventricle.

In other examples a preset for a needle visualization procedure may be employed wherein the handheld device 108 overlays a B-mode ultrasound image, generated using parameters set for visualizing an inserted needle, on top of a B-mode image obtained with parameters set for normal vascular imaging. Still other presets may be employed and the preset employed will typically depend, at least in part, on the imaging procedure the clinician is conducting and the clinician's choice of preset.

The menu 114 may include text boxes that display text representing the preset used during the ultrasound imaging and that the images are displayed with a biplane view. Biplane views may be understood as displays of two planes of imaging, one along the longitudinal axis of the probe and the other along the transverse axis of the probe. In the depicted example, the longitudinal axis view is displayed on the bottom of the screen and the transverse axis view is displayed on the top of the screen, to provide a perpendicular plane of view of the patient lumen.

In any case, FIG. 1 illustrates that the system 100 allows the clinician to carry out an ultrasound imaging study of a patient. The study may involve a vascular access, cardiac valve function, pre-natal observations, lung capacity or any other study that will benefit from ultrasound imaging. The clinician can select a preset suited for the study and the preset will have parameters, such as depth of penetration and frame rate, that the imaging device system will use to carry out the imaging.

In the embodiment depicted in FIG. 1, the application 109 may include a neural filter module that operates to transform image data generated by the probe 102 so that the distribution of the images presented to the clinician matches, or substantially matches, the distribution of images generated by a cart-based ultrasound imaging probe. In this way, the application 109 applies the neural filter to generate a transformed image, or stream of images, wherein the transformed images have the look and feel of the type of images generated by cart-based ultrasound imaging probes. Thus, a clinician more familiar with cart-based ultrasound imaging systems can activate the neural filter and thereby be presented within window 110 with images that are more familiar in look and feel and that provide sharper and cleaner images. Moreover, the sharper features and a cleaner, less cluttered image generated with the active neural filter may help clinicians find the anatomical structure of interest more quickly and allow the clinician to make a more accurate and faster diagnosis. In this embodiment a neural filter can be understood to encompass, but is not limited to, an AI system trained to take image data, or a stream of image data, and process it in such a way that it can output image data or a stream of image data which when processed by a standard image processor, produce visual images which are in a different visual style as defined by common properties such as resolution, sharpness, and noise, without losing the essential components of the original image. For example, if you trained an AI system to act as a neural filter which translates photographs into images which resemble Rembrandt oil paintings, this system would be able to take a photograph of a sunset and generate as output an image which looks like a Rembrandt oil painting of a sunset, the original content of a sunset is still represented in the output image, but the style of the image now resembles Rembrandt's style of painting in that shapes of individual elements may be altered or the color palate may be different. Additionally, such filtered images may increase the ease and efficiency of the clinician locating the probe 102 on the habitus of the patient. As used herein the term cart-based ultrasound imaging device encompasses, but is not limited to, ultrasound systems of the type that are typically large enough to be carried on a cart and powered by wall power, and having an image or video display that is mounted to the cart to allow the clinician to move the ultrasound probe while viewing the display. However, it will be understood by those of skill in the art that the systems and methods described herein are not limited to any specific set of cart-based imaging devices or systems. Moreover, it will be understood by those of skill in the art for the systems and methods described herein that the term cart-based ultrasound imaging device will encompass any ultrasound imaging device that generated images that were used, or collected for use, as a part of a training set of images employed to train the neural filter applied by application 109.

In certain embodiments, the application 109 responds to controls entered through the UI controls of window 112. For example, the user interface window 112 may include a control, typically referred to as a widget, that is presented as an icon within the user interface window 112. The clinician can activate the widget to direct the application 109 to activate the neural filter for processing images presented within window 110. In certain embodiments, the widget maybe a preset selection widget that allows the clinician to configure the system 100 for certain preset operations. For example, a preset control may be presented within window 112 to allow the clinician to select preset parameters for a cardiac imaging procedure. In this embodiment, the preset control acts as a user interface to instruct the application 109 to control operation of the neural filter. A preset control, or preset configuration, includes but is not limited to, a set of parameter values within a larger system's capabilities which are configured to specific values which optimize or improve the performance of the overall system for a specific purpose and having those values grouped together into a single selectable setting for the overall system. Typically the parameters which are a part of the preset can be set individually and it would be possible for a user to manually set each parameter value to the same level that they are in the preset. The benefit of the preset is that the combination of parameter values are optimized for a specific use-case of the system and where that use-case is common. That allows the user to save time by setting all parameter values to the optimal level for a common use-case all at once. It will be clear to those skilled in the art that in other embodiments a preset control may encompass other aspects depending on the application at hand. The preset control therefore acts as a UI switch that activates the neural filter and causes the application 109 to load parameters associated with the cardiac preset controls. In this embodiment, it can be understood that a UI switch may encompass to a button or selectable digital icon which is incorporated into the User Interface of a system or tool which allows the user to toggle on and off the feature associated with the button or digital icon. It will be clear to those skilled in the art that in other embodiments a UI switch can encompass other forms which are suited to the application at hand. Typically, such parameters are set to optimize the system 100 for imaging cardiovascular anatomy of the patient. In this embodiment, the preset parameter configurations may include the activation of the neural filter of application 109. As such, the application 109 will transform images collected by probe 102 to have an image distribution associated with cart-based images. In this embodiment the image distribution can be understood as a set of images which are of the same type based on fitting a pre-determined categorization but which have differences in their visual characteristics and content as to maintain their membership in the category and to define the range of the distribution. For example, if you have a set of images which are all ultrasound images of the heart, those images will not all be identical to each other. Variations in patient anatomy, probe placement, device strength and other factors will create differences between the images, however all of these images can still be categorized as of the same type since they are all ultrasound images of the heart. It will be clear to those skilled in the art that in other embodiments image distribution may encompass other elements based on the application at hand. The transformed images will be presented within window 110 for use by the clinician during the cardiac imaging operation. In one embodiment, the clinician may have in additional control within user interface window 112 which allows the clinician to toggle the use of the neural filter within application 109 from active to inactive. In such embodiments, the clinician may activate the neural filter by selecting the cardiac preset user control within window 112. As the clinician moves and orients the probe 102 over the habitus of the patient, the clinician may achieve a location and orientation the clinician deems proper for collecting images for a cardiac imaging procedure.

In some practices, the clinician will generate images for the imaging procedure with the neural filter active and operating as discussed herein. The clinician will locate and orient the probe 102 on the patient's habitus and capture and filter images. The filtered images may be employed during the diagnostic process. This process may include taking measurements of organ characteristics, such as ejection fraction of the patient's heart, chamber volume, hypodermic needle placement and depth relative to a patient's vein, lung volume and other measures and observations that the clinician may employ during diagnosis and treatment. Thus, the clinician may use the images filtered by the neural filter to position the probe 102 and collect images and the filtered images may be analyzed by the clinician as part of the clinical treatment process. Optionally and alternatively, once the probe 102 is located and oriented at a selected location on the patient's habitus, the clinician may employ the user control within window 112 to toggle the application 109 to deactivate the neural filter. Once deactivated, the application 109 will present within window 110 the image data that is collected by the probe 102 without transforming such image data to cart-based image distributions. In this way, the clinician may employ the neural filter of application 109 during the location and orientation of the probe 102 to facilitate easy and efficient location and orientation of the probe 102 relative to the habitus of the patient. Once so located, the clinician may use the UI to deactivate the neural filter of application 109 to collect images that are ground images, that is representative of the image data collected by probe 102 without transforming such data to achieve the look and feel of cart-based imaging devices.

In alternative embodiments, the application 109 may respond to the UI 104 on probe 102. In this way the clinician may manually toggle the UI switch 104 to direct the application 109 to activate or deactivate the neural filter that may be filtering image data generated by probe 102. Those of skill in the art will recognize other ways to activate or deactivate the neural filter of application 109 so that the clinician has control over whether the neural filter is being applied to image data generated by probe 102.

In FIG. 1 the application 109 may operate the handheld device 108 to display an icon 120 within menu 122. The icon 120 may present a color, such as red or green, that indicates to the clinician whether the neural filter of the application 109 is being applied to image data generated by the probe 102. In other embodiments, a text message may appear within the window 110 to indicate whether the neural filter is active or inactive. Still other embodiments for indicating to the clinician the activity status of the neural filter may be used with the systems and methods described herein without departing from the scope of this disclosure.

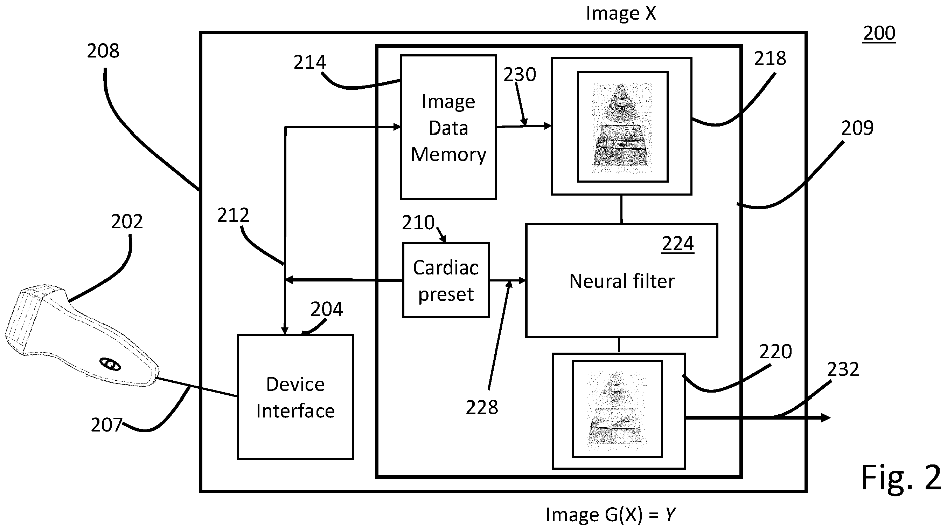

FIG. 2 depicts pictorially a system 200 that has a handheld device 208 that supports executing application program 209. The application program (or application) 209 may receive image data from the probe 202 and transform that image data into image data that has a distribution that matches or substantially matches the distribution of images generated by cart-based ultrasound imaging devices. In particular, FIG. 2 depicts a probe 202 that connects via a data path 207 to a device interface 204. The device interface 204 couples to a bus 212 and that bus 212 connects to a preset module 210, which in this example is the cardiac preset module. The bus 212 also connects to an image data memory 214. The application program 209 can store image data from the probe 202 and received through the device interface 204 in the image data memory 214 and access that image data in the memory 214 via the data path 230. The application 209 may store portions of the received image data in the memory buffer 218. The portion of the image data in the buffer 218 may be, as discussed below, filtered with the neural filter 214. In some embodiments, the portion of image data stored in the buffer 218 corresponds to a frame of image data within a video. As further shown, the application 209 includes a neural filter 224. The neural filter 224 in FIG. 2 is depicted as a functional block diagram that is located within and is part of the application 209. The neural filter 224 can access image data from the memory buffer 218 and can transform that data and store the transformed image data in the memory buffer 220. The transformed image data, as described above, includes an image distribution that matches or substantially matches the image distribution of cart-based ultrasound imaging devices. That transformed image data from memory buffer 220 may be delivered via data path 232 to a video display, such as display 110 of FIG. 1, for presenting the transformed image data to a clinician. It can be understood that the term image data includes, but is not limited to, a set of underlying digital data which when processed by a traditional computer processor, produces an image whose characteristics and defined by the data. It will be clear to those skilled in the art that in other embodiments the term image data can encompass other elements based on the application at hand.

The application 209 may be a computer program executing on a processor built within the circuit board of the handheld device 208 that couples to the probe 202. The development of such applications that execute on a handheld device such as a smartphone or tablet and that carry out the depicted functions of the application 209 is well known to those of skill in the art. Techniques for developing such applications are set out in for example, Alebicto et al., Mastering iOS 14 Programming: Build professional-grade iOS 14 applications with Swift 5.3 and Xcode 12.4, 4th Four ed.; Packt Publishing Ltd (2021). For clarity and ease of illustration, the application 209 is depicted as a functional block diagram. The functional blocks of application 209 include cardiac preset 210, image data memory 214, memory buffers 218 and 220 and the neural filter module 224. The handheld device 208 also includes a device interface 204, also depicted as a functional block, that interfaces with the probe 202 to allow the exchange of data between the probe 202 and the handheld device 208. The device interface 204 in one embodiment, is a conventional electronic transceiver circuit capable of transmitting and receiving data over a wire connection, such as the depicted data path 207. Additionally, the device interface 204 couples to the bus 212. The bus 212 maybe a typical data bus used on circuit boards to exchange data among different functional modules on the circuit board. In the depicted embodiment the device interface 204 couples to the data bus 212 to exchange information with the cardiac preset module 210 and the image data memory 214. The cardiac preset module 210 may be a module, in some embodiments connected and responsive to a UI widget, and capable of delivering data to the probe 202. In one embodiment, the cardiac present module 210 of the application 209 detects that the clinician has selected this preset. This selection may occur through any suitable process, and in one particular embodiment, the selection occurs by the clinician selecting the cardia preset option from a menu displayed on a screen of the handheld device 208, as described with reference to FIG. 1. The cardiac preset module 209, in one embodiment, may retrieve from a memory device on the handheld device 208 those parameters that are associated with the cardiac preset. Alternatively, in other embodiments those cardiac preset parameters may be stored in the cardiac preset module 209 upon activation of the application 209. In any case, the cardiac preset module 209 has the parameters for configuring the probe 202 to operate as established for this preset and the application 209 transmits those parameters across the bus 212 to the device interface 204 for delivery to the probe 202. The probe 202 is thereby configured according to those parameters. Optionally,, the data from the preset module 210 are parameters that adjust the image processing being carried out by the application 209 as well as for setting parameters for the operation of the probe 202. In the depicted example, the preset module 210 is a cardiac preset module that is capable of configuring the probe 202 to operate with certain parameters such as power parameters, correction angles, and other similar parameters often used in the ultrasound imaging of a patient's heart. Additionally, the cardiac preset module 210 may include a parameter for activating the neural filter 224 and to this end the cardiac preset module 210 is depicted as connected via data path 228 to the neural filter 224 of application 209. In one embodiment, the cardiac preset module 210 may activate an interrupt signal that can be detected by a processor executing the application 209 to direct the application 209 to activate or deactivate, depending on the state of the neural filter, the operation of the neural filter 224.

Additionally, the device interface 204 communicates via the bus 212 with the image data memory 214. The image data memory 214 may be a data memory allocated and maintained by the application 209 and capable of storing image data generated by the probe 202. FIG. 2 pictorially depicts the image data memory 214 as capable of delivering image data via path 230 into the memory buffer 218. In this way, raw image data from the probe 202 may be stored in the data memory buffer 218 of the application 209. Typically, such a memory buffer 218 is a software allocation of memory space dedicated to certain type of data structure, such as an image frame. In operation, the neural filter 224 of application 209 may access data in the memory buffer 218 to transform that stored raw data in memory buffer 218 into image data capable of being stored in memory buffer 220. The transformed data stored in memory buffer 220 may have an image distribution that matches, or substantially matches, the image distribution of images generated by cart-based ultrasound devices.

In one embodiment, the neural filter 224 is a software module executing as part of the application 209. The neural filter 224 in this embodiment is a software module capable of translating the style associated with one set of images, in this case images generated by probe 202, to the style associated with another set of images, in this case images of the type generated by cart-based ultrasound imaging systems. As such, the neural filter 224 may be understood as an image-to-image translator that converts an image from one representation of a given scene, x, in this example the scene will be an anatomical image rendered by the ultrasound imaging probe 202, to another representation, y. As used herein, translating image data can be understood to encompass, but is not limited to a process of altering the data which define a visual image in such a way that the original content of the image is largely maintained, but that the resulting translated image resembles a different category of images enough that a person familiar with both the original and translated categories would recognize the translated image as being a part of the translated category. For example, translating the image data of an ultrasound image of a heart taken by a handheld ultrasound imaging device into an image of the type produced by cart-based ultrasound systems would mean altering the image data which define the image from the handheld device such that the image produced by the translated image data would look like a typical ultrasound image of the heart taken by a cart-based ultrasound system to a knowledgeable clinician. It will be apparent to those skilled in the art that in alternate embodiments translating image data may encompass other elements depending on the application at hand. As discussed below, the neural filter 224 can process image data from the probe 102 to adjust an output distribution or output distributions of the image data to conform with an output distribution of image data generated by cart-based ultrasound systems and to maintain the content of the image data that had been generated by the probe 102. Thus, the neural filter 224 may maintain the content of the ground images generated by the probe 102, while processing those ground images to achieve output distributions of cart-based images and thereby for example, achieve the sharpness and clarity of images associated with cart-based ultrasound devices. In this embodiment, output distribution may be understood as a set of images which have been processed by a neural filter which while having enough similarity as to categorize them as of the same type, do have some variations in their image content and visual properties which define the range of the distribution. For example, if a neural filter was given a set of photographs of a house, and was tasked with translating them into a images in the style of a newspaper comic strip, the output distribution of the neural filter would be a set of images which all depicted houses, with rooves, doors, windows, etc. which generally aligned with the corresponding elements of the original photographs, but the elements will have been altered possibly by changing the color palate or rounding the edges of sharp corners, so that the houses are still recognizable as houses, but would not look out of place in a newspaper comic strip. It will be clear to those skilled in the art that in other embodiments output distribution may encompass other elements based on the application at hand.

In one embodiment, the neural filter 224 may be implemented as an Application Specific IC (ASIC), a Field Programmable Array (FPGA), a micro-controller, a software module executing on a microprocessor or any other implementation capable of being configured to carry out the image-to-image functions described herein. In one particular embodiment, the neural filter 224 is a software module executing on the microprocessor on the handheld device 208.

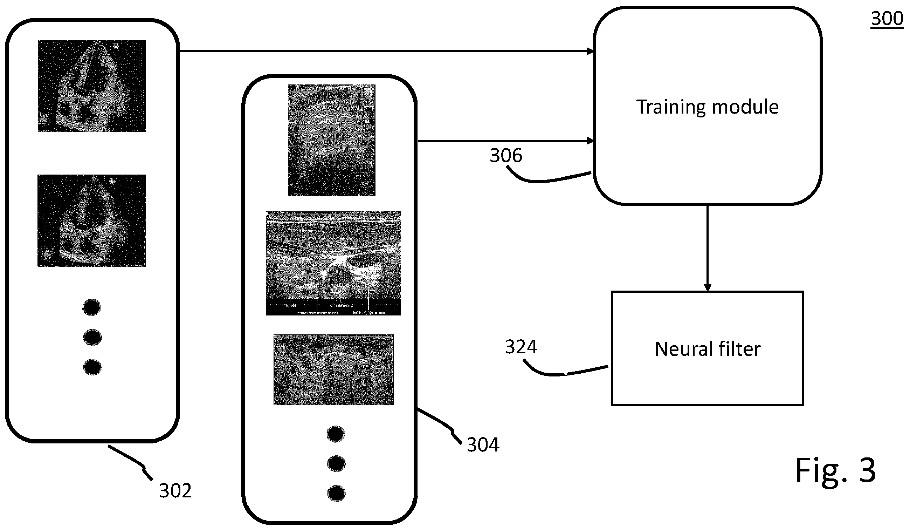

FIG. 3 depicts pictorially one example system 304 for creating a neural filter 324 of the type used in the system of FIG. 2. The system 304 employs two data sets of images and a training module to create the neural filter 324 by generating and storing within the neural filter 324 a mapping function that is capable of translating images in a first domain to images of a second domain. In general, the image data sets 302 and 304 will act as the input data and the output data that the training module 306 will employ as it implements a back-propagation process for adjusting the weightings of the neural network being trained. In this embodiment the training module can be understood to encompass, but is not limited to, a set of procedures performed on an AI system so the AI system is able to accurately identify, categorize, and generate data which are inputted and outputted from the system. These procedures may include showing the system cataloged data which overtly identify the data so that AI can accurately recognize it in the future, reviewing the output of an AI system for accuracy and providing feedback of the accuracy to the system, having the AI attempt to disguise it's generated content as original content for an adversarial function which attempts to discern AI generated content from original content. It will be apparent to those skilled in the art that in alternate embodiments a training module can encompass other elements depending on the application at hand.

In particular, FIG. 3 shows a system 300 that includes a first set of images 302, a second set of images 304, a training module 306 and a neural filter 324. The first and second set of images 302 and 304 are, as shown in FIG. 3, anatomical scenes generated by ultrasound imaging. The first set of images 302 may be stored in an image memory and the second set of images 304 may be stored in a different image memory. In either case, image data generated from a portable ultrasound imaging device, such as the probe 102 depicted in FIG. 1, may be stored in an image memory to create the data set 302 and images from a cart-based ultrasound imaging system may be stored in an image memory to create the second set 304. These image sets 302 and 304 provide the inputs and outputs the training module 306 will employ to train the neural network to be capable of performing the image-to-image translation used to give images generated by the handheld ultrasound system the "look and feel" of images generated by cart-based ultrasound systems. This trained neural network can be realized as a translation mapping function and that function can be instantiated into the neural filter 324 so that the neural filter can perform this translation function. In this embodiment a mapping function can be understood as encompassing, but not limited to, a piece of computer code, which in this instance, works within an AI system, to process data and determine what broader categories that data fits within. For example, it can review the image data which describes an ultrasound image to determine if the sharpness characteristic of that image more closely matches the typical sharpness of images produced by handheld, or cart-based ultrasound systems. In addition, when training the AI system, mapping functions can review input data and identify what data of a data set the input data would correspond to if it were to comply to the other set's categorization requirements so that the AI can learn to define how characteristics of an input would translate to fit the requirements of another category.

In one embodiment, the neural filter 324 may include a translation mapping function developed by a training module 306 that carries out an analysis of supervised settings, where example image pairs {x, y} are available. These techniques may be employed to create a training module 306 that is suitable for use with paired image data. In such an example, the images in the first data set 302 and the second data set 304 are paired. A paired data set is a data set where an image in the first data set has a corresponding respective image in the second data set. Typically, that corresponding image includes essentially the same anatomical scene as depicted in the respective image of the first data set. In this embodiment, the training module 306 employs a training process suitable for use with paired image data, such as the training process disclosed in J. Johnson, A. Alahi, and L. Fei-Fei.; Perceptual losses for real-time style transfer and super-resolution; In ECCV, pages 694-711; Springer (2016). Such training modules 306 may be software modules that typically implement pixel based image comparisons between respective images of a pair of images. The training module 306, based on these comparisons, develop a translation map for translating images from one domain to the other. The developed translation map may be supervised, which is essentially a testing process to determine the accuracy and robustness of the translation mapping function. Such supervision may occur by using known pairings of respective images between the sets to ensure that the developed translation map translates images from one domain to the other without introducing unacceptable losses and inaccuracies in the content of the translated image, and may ensure that errors in style do not occur, such as failures that increase noise, such as line fuzziness, into the translated image. The term noise can be understood to encompass but is not limited to a visual characteristic of an image which defines the amount of visual information displayed in the image which may distort, transform, block, or add to what is seen in the image compared to the reality which the image is meant to capture. It will be apparent to those skilled in the art that in other embodiments this term may encompass other elements based on the application at hand.

In alternate embodiments, the neural filter 224 may include a translation mapping developed by a training module 306 that works from unpaired input-output examples. In this embodiment unpaired images can be understood to include but are not limited to, images of different categories which are only identified by their category, and not the relationship between each other. For example if two sets of ultrasound images are differentiated by one set being from handheld, and the other from cart-based ultrasound systems, no other identifiers will be added to the images even if there are images in both sets which depict the heart, liver etc. Still other embodiments unpaired images may encompass other elements without departing from the scope of this disclosure. Such techniques are disclosed in, for example Zhu et al.; Unpaired Image-to-Image Translation using Cycle-Consistent Adverserial Networks; arXiv: 1703.10593 (2020) were employed. For such embodiments, supervision of the developed translation may be provided at the level of sets of images. In these processes that employ unpaired data, the development of the neural filter 324 includes collecting a first set of images in Domain X, which in this case will typically be images generated by a handheld device of the type depicted in FIG 1. Further, the process will collect a second set of images in different domain, Domain Y. For the neural filter 324, the Domain Y may be a set of images of similar anatomical image scenes as in Domain X, although not paired images, and may be generated by cart-based ultrasound imaging devices. Thus, for example, Domain Y may include anatomical images of patients captured by cart-based ultrasound imaging devices. It will be recognized by those of skill in the art that both Domain X and Domain Y will typically include images of anatomical scenes of patients. There will therefore be similarities between these sets of images of Domain X and Domain Y even though they were generated using different types of ultrasound imaging systems. However, differences in the characteristics of the images in Domain X may exist between the characteristics of images in Domain Y. For example, the contrast, saturation, grayscales, aspect ratios, and other image characteristics may differ in distribution between the images of Domain X and the images of Domain Y. Other differences in image characteristics, which are less readily identified by human users, but can be found by machine learning as the training module 306 trains the neural net, may also be considered by the systems and methods disclosed herein. In any case, these differences in image characteristics between handheld and cart-based systems may make it more difficult for a clinician accustomed to working with cart-based ultrasound imaging devices to quickly put to use a handheld ultrasound imaging device. To address this problem, the system 300 employs the image data sets 302 and 304 and the training module to develop a mapping function that can be incorporated as the neural filter 324. The neural filter 324 can be used by the application program 209 to translate images generated by the handheld ultrasound device 100 into images that have characteristic distributions that match a cart-based device's distribution.

In operation, the system 300 delivers the images from image sets 302 and 304 into the training module 306. The training module 306 trains a neural network to identify and analyze characteristics of the images related to image set 302, and characteristics of the images in the image set 304. Distribution information about these characteristics is developed for the input data set 302 and for the output data set 304. The training module develops a mapping that maps images of the type from the first data set 302 to the domain space associated with images of the type in data set 304. In particular, the translation map developed by the training module 306 maps images of the type from the image set 302 such that translated images from set 302 will have image distributions that match, or are similar to, the image distribution characteristics of images of the type found in data set 304. In this way, images captured by the handheld device can be translated to have the distributions found in the Domain of images generated by cart-based devices. This will give the translated images a look and feel that is highly correlated to the look and feel of images found in the second data set 304. This high correlation is such that a process of discriminating translated images from native images within the set 304 is difficult for the human observer. In one embodiment, discriminators are applied to challenge both generative functions. The discriminators will aim to distinguish between ground images from a Domain, whether X or Y, and images that have been translated into the respective Domain. The use of discriminators can increase the match between distributions of ground images of one Domain with images translated into that Domain. The test processes employed to discriminate translated images from actual images from the Domain may include human visual inspection or machine inspection that analyses image characteristics such as distribution of contrast, image noise and other criteria. It will be apparent to those of skill in the art that any suitable criteria may be employed, and the inspection criteria for discriminating between translated images and ground images originally from Domain will typically depend upon the application being addressed. The discriminator criteria for convergence may, in one example, be set such that the discriminator has an accuracy of about fifty percent (50%) on both domains and the discriminator cannot distinguish effectively between the translated images and the ground images. This fifty percent level, meaning the discriminator is correct or incorrect in determining for instance that a translated image is actually a ground image, indicates that the generative network is generating translated images with data distributions that the discriminator cannot distinguish from the ground images.

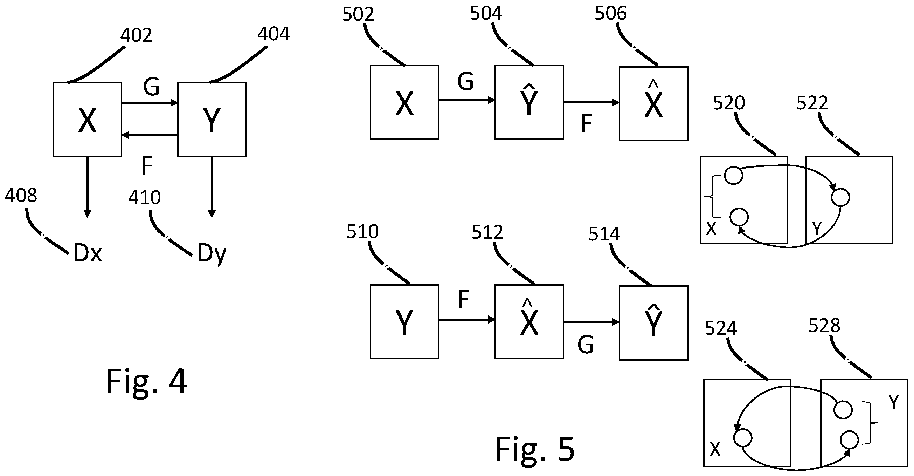

FIGS. 4 and 5 depict that for one particular embodiment for generating a neural filter 224 from unpaired image sets, the training module 306 may apply a process that sets as objectives a cycle consistency loss value and an adversarial discriminator. Specifically, FIG. 4 depicts that the training module 306 may include two generative models G and F. The generative model G may be used to translate image data from the X Domain to the Y Domain, and the F generative model may be used to translate images in the Y Domain to the X Domain. This is shown in FIG. 4 by the direction of the respective arrows indicating the translation operations of G and F, 402 and 404. FIG. 4 further illustrates by the opposing directions of the respective arrow that the operations G and F together yield the ability to translate image data cyclically, which means translating image data back and forth between the two Domains. FIG. 4 further shows that there are adversarial discriminators Dx and Dy. In this particular embodiment, the training module 306 applies adversarial losses to both generative functions, G and Y. The adversarial loss can test, typically by testing the match over the tested images, the distribution of generated images to the data distribution in the target domain. In this particular embodiment, the generative models G and F are pitted against an adversary, which is the respective discriminative models Dx 408 and Dy 410 that learn to determine whether a sample is from the translated image data or the original data distribution, such as the cart-based images from image data set 304. The generative models F and G are, in essence, attempting to disguise the images from Domain X as images from Domain Y, and to disguise images from Domain Y as images from Domain X, by finding a translation mapping that avoids detection. To force the generative models F and G to operate effectively, that is to disguise the image well, discriminative models are used that try to detect the translated images. To that end, the discriminators will detect translated images and will use information about the detected translations to maximize the areas of distinction between translated images and images originally from the respective Domain. Competition between the generative model and the discriminator drives improvement with the accuracy and robustness of the translation mapping function. See Generative Adversarial Nets, Goodfellow et al.; Part of Advances in Neural Information Processing Systems 27 (NIPS 2014).

FIG. 5 depicts that for this particular embodiment, there is a cycle consistency loss to reduce the likelihood that the learned mappings G and F will contradict each other. This is shown pictorially in FIG. 5. In particular, FIG. 5 shows that the translation from the X Domain 502 by the mapping function G to the Domain 504 can subsequently be translated from the Domain 504 to the Domain 506 by application of generative function F. Similarly, going in the opposite direction, FIG. 5 also shows that translation from the Y Domain 510 by application of generative function F leads to the translation of images into Domain 512, which can subsequently be converted by application of generative function G to the Domain 514. In each of these cases a cycle consistency can be measured. The cycle consistency is shown pictorially by the blocks in FIG. 5 such as block 520 and 522 that shows a mapping from the X Domain to the Y Domain and that there is some gap between the two X Domain images that arises when both G and F are applied. That gap indicates a loss in accuracy because the cycle is not perfectly complete and fails to close without any gap between the original (source) image of the X domain 520 and cyclically translated image of that original image, also shown as an image in the X Domain 520. Thus, functions G and F are not capable of perfectly translating between Domains X and Y. This is shown by the separation in Domain 520 that exists between the original image and its cyclically translated version also in Domain 520. This gap represents the cycle consistency loss. A similar gap is shown for Domain 528. Techniques for developing these cycle GAN, cycle consistent adversarial networks, are disclosed in Zhu et al.; Unpaired Image-to-Image Translation using Cycle-Consistent Adverserial Networks; arXiv: 1703.10593 (2020), the contents of which are incorporated by reference. In this embodiment a cycle consistent adversarial network can be understood to include, but is not limited to, a program which tests the accuracy and quality of an AI system by evaluating the content which is lost or added when the AI alters the data, and the accuracy of the alterations to the Al's goal. For example, if the AI takes a set of landscape photos, and alters them to appear like an abstract oil painting, the network may evaluate the system's accuracy by reviewing a set of images which contain both AI outputs, and real abstract oil paintings and guessing which of the images were produced by AI. If the network is correct nearly 50% of the time, then the AI has been able to produce images which cannot be distinguished from ground images better than randomly guessing, and is deemed to be accurate. Furthermore, an AI system may translate a landscape photo into an image in the style of an abstract oil painting, then translate that output back into the style of a landscape photo. A network may then review the original photo compared to the final retranslated AI output and compare the results. The larger the differences between the original and translated images, the less content-consistent the AI's translation process is deemed to be. An adversarial network will perform the testing procedures such as those described above until the accuracy and content-consistency reach pre-determined levels of acceptability, at which point the AI is deemed to be successfully trained. The development of the generative functions G and F may be achieved using deep learning techniques. Such deep learning techniques may be employed to identify characteristics of images in the Domains X and Y and the distribution of those characteristics in each of those Domains. In such deep learning embodiments, the neural filter 224 may match image distributions for image characteristics identified through deep learning between images in Domain X translating those distributions into distributions that match or essentially match those found in Domain Y. The statistical differences between these sets of distributions can be used to translate the style of images in Domain X, to the style of images in Domain Y, while maintaining the content of the images as the images are translated between domains. The result is an image, generated by probe 202 and having the content captured by the probe 202, but rendered with output distributions that match, or substantially match, the distribution of Domain Y and thus with a style matching the style of cart-based ultrasound imaging devices.

The mapping developed by the training module 306 may then be loaded into a neural filter module 324. In one embodiment the training module 306 loads the map into the neural filter 324 as a portion of a neural network capable of translating images from the first domain associated with the set of images 302 into images having a distribution that matches images in the data set 304. Alternatively, the training module 306 may develop look up tables, a series of functions, or any other suitable mechanism for storing the translation map developed by training module 306 into the neural filter 324. In one optional embodiment, the systems and methods described herein employ two stages of training to develop a training module 306 that is of size more suited for use in a handheld device. The first stage of this two-stage training is as described above, and the training module developed may be referred to as a "teacher model". The two-stage training employs a second stage for the purpose of processing the teacher model to develop a student model that is effective for the application at hand, which in this example is translating the data distribution of ultrasound images. However, the student module will be smaller in size than the teacher model and therefore more readily installed in an application executing on a handheld device and may be more able to execute on the handheld device as a real-time application.

To this end, in one embodiment the teacher model is distilled into a smaller student model having a model architecture that runs in real-time on the handheld device. Techniques for such a distillation process are known in the art and include those disclosed in or similar to those disclosed in Chen et al.; Distilling Portable Generative Adversarial Networks for Image Translation; The Thirty-Fourth AAAI Conference on Artificial Intelligence; pages 3585-3592; (2020). In one embodiment, the distillation process includes sampling a source image and running it through the teacher model to generate the teacher target image. Additionally, the distillation process will run that source image through a student model and generate an output. Optionally, the student model may be size constrained. For example, the student model may be constrained to have half or quarter channels of the teacher model. This can reduce the size of the student model as compared to the teacher model. Thus, in these embodiments, the student model generated output may be generated by a size constrained student model.

The distillation process will reduce the distance of the student model generated output to teacher target using a suitable technique such as, but not limited to, pixel-wise mean absolute distance measures. The distillation process will update the discriminator that provides that the student model generated outputs have data distributions from the target domain. The smaller student model may be employed as the training module 306.

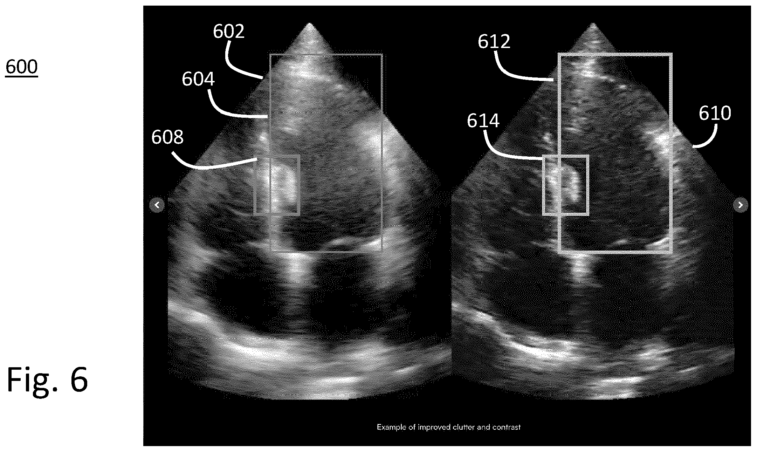

FIG. 6 depicts a first image rendered in a side-by-side presentation with a ground image on the left and an opposing translated image on the right generated by application of a neural filter of the type described with reference to FIG. 3. In particular, FIG. 6 presents an image 600 where the left-hand side is an image generated by a handheld system such as the systems of the assignee hereof, and a right-hand image that presents that image after translation by application of a neural filter, such as the neural filter disclosed with reference to FIG. 3. The left-hand image in FIG. 6 depicts an image 602 collected and generated during a cardiac procedure. Image characteristics of the type commonly used to evaluate and characterize an image include clutter and contrast. FIG. 6 includes a window 604 that shows a cavity within the cardiac image 602. Image 602 further includes a window 608 that shows a cardiac feature within the cardiac image 602. The image 602 and the features of the cavity and the cardiac feature within windows 604 and 608 respectively illustrate the contrast and clutter provided by an image generated by the handheld device. This image 602 is the ground image for the neural filter process described herein. The right-hand image 610 also includes two windows, 612 and 614 each window showing the same respective features as in the image 602. The image 610 is a translated image representative of the ground image 602 after filtering by a neural filter. The window 612 shows the cavity of image 610 and illustrates a reduction in clutter within the translated image of the cavity. The window 614 depicts the cardiac feature shown in window 608 of the ground image. Window 614 illustrates a reduction in clutter in the image of this feature as compared to in the ground image 602. Both windows 612 and 614 and the image 610 overall show a reduction in clutter and an increase in contrast. The aesthetic of the image 610 provides the look and feel of an image generated by a cart based ultrasound device. This aesthetic is created by the neural filter translating the ground image 602 to have image distributions aligned with the image distributions generated by cart-based devices. As illustrated in FIG. 6 this may present a translated image 610 that has less clutter and greater contrast than the ground image 602 generated by the handheld device.

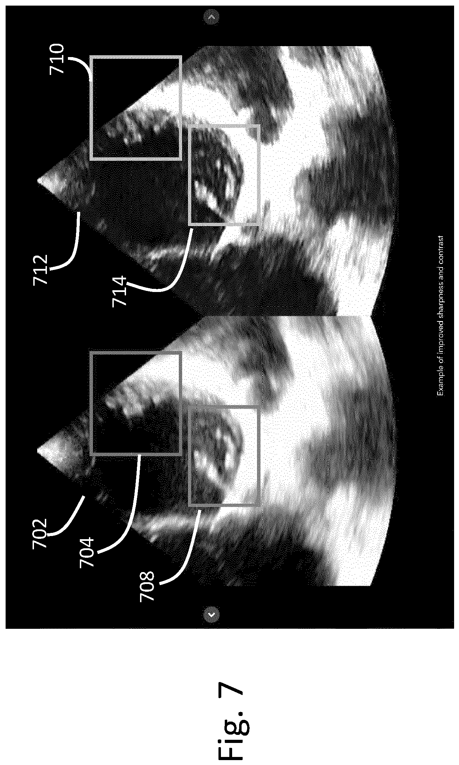

FIG. 7 depicts a second image rendered in a side-by-side presentation with the ground image on the left and the translated image on the right and having been generated by application of a neural filter of the type described with reference to FIG. 3. In particular, FIG. 7 depicts left-hand image 702 that is a ground image and a right hand image 712 that is a translated image of the ground image 702 wherein that translation results from the application of a neural filter such as a neural filter described with reference to FIG. 3. In particular, image 702 includes two windows 704 and 708 both of which highlight features of the image 702, which in this case is a cardiac image generated by a handheld device such as the handheld devices manufactured and sold by the assignee hereof. The image 702 has a sharpness and contrast, where sharpness and contrast are understood as conventional image processing characteristics used to evaluate and characterize generated images. The right-hand side 712 is the translated image of the ground image 702, wherein 712 is generated by application of a neural filter as described herein to the ground image 702. Image 712 includes windows 710 and windows 714 each of which highlight the respective features shown in image 702 by windows 704 and 708. As can be seen by a comparison of the cardiac features in windows 710 and 714 versus windows 704 and 708, the translated image 712 is rendered with an increased sharpness and contrast. The term sharpness as it is used in this embodiment can be understood as A visual characteristic of an image which defines the clarity of the boundaries between individual elements within the image such that an image with clearer boundaries between its individual elements is categorized as having a higher level of sharpness. It will be clear to those skilled in the art that in other embodiments sharpness can encompass other elements based on the application at hand. The image 712 is a translated image generated from the ground image 702 and translated to have distributions in line with the distributions generated by cart-based ultrasound devices. Thus, image 712 has the look and feel and aesthetic of an image generated by a cart-based device.

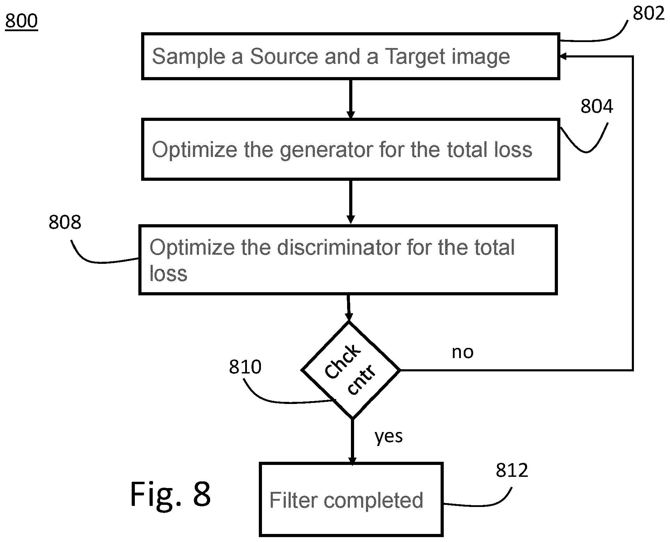

Turning to FIG. 8, a process 800 is depicted for generating and building the neural filter of the type that can be used with the systems and methods described herein. The process 800 begins at 802 where a source and target set of images are sampled. In this particular example, the source images being sampled are images generated by handheld ultrasound systems and the target images being sampled are images generated by cart-based ultrasound images. The process 800 proceeds to 804 where the process 800 will optimize or substantially optimize the generative functions F and G for the cycle consistency loss as images are transformatively cycled from the first domain to the second domain and then from that second domain back to the first domain. The generative functions F and G are optimized for the measured cycle consistency loss. The process 800 then proceeds to 808 where the discriminator functions Dx and Dy for each of the generative functions F and G are optimized for the adversarial loss. After passing through the system a first time the process 800 can proceed to the counter check 810 where a counter is checked to see whether or not enough sufficient samples have been made. In this embodiment, the counter may be set to a predetermined number to ensure that the process samples that predetermined number of sample images. If not the process 800 will proceed back to 802 for additional sampling and optimization. If sufficient samples have been taken, the process 800 may proceed to 812 where the filter is deemed complete and is ready to be loaded into and made part of the neural filter. In alternative embodiments, different criteria may be applied in the decision action 810, such as having a checkpoint based certain measures of success, such as meeting a goal for the cycle consistency loss.

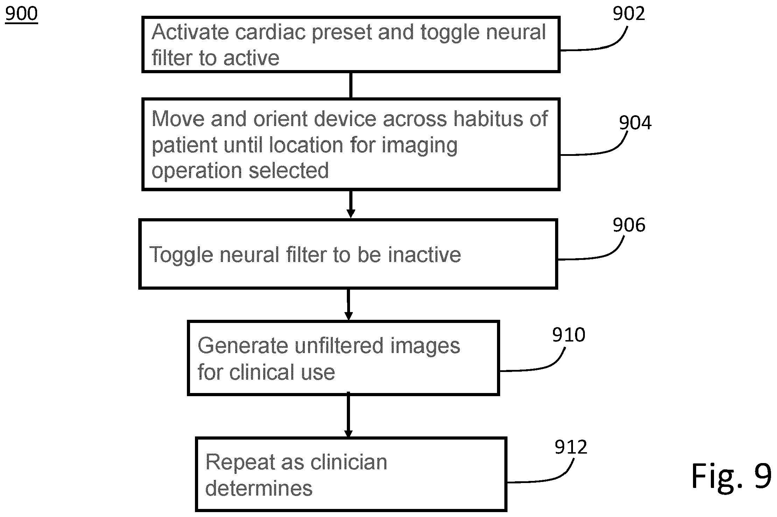

FIG. 9 depicts another embodiment of the systems and methods described herein. In particular, FIG. 9 is a flow chart of a process for using a system, such as the system 100 depicted in FIG. 1. In particular, the process 900 begins at 902 when the clinician activates a preset such as a cardiac preset and activation of the cardiac preset toggles the neural filter of the system to be active. The process proceeds to 904 wherein the clinician can move and orient the probe of the ultrasound device across the habitus of the patient until a location and orientation for imaging has been selected. The depicted process 900 may then proceed to 906 wherein, in this embodiment, the clinician toggles the neural filter to be inactive. This deactivates the neural filter and causes image data generated by the probe of the handheld ultrasound device to be presented to the clinician directly. This is shown in 910 wherein the system generates unfiltered images for clinical use. After reviewing images, the clinician at 912 may choose to reactivate the neural filter and relocate and reorient the probe or determine that the imaging procedure has been successfully completed. In alternative practices, the process 900 after 904 may then proceed to generate images with the filter remaining active for the clinician to use for diagnosis and for treatment. This alternate practice maintains the neural filter in an active state and causes filtered image data to be presented to the clinician. In this alternative practice, the clinician may use the filtered images which may be sharper and have higher clarity than unfiltered ground images, during diagnosis, measurement and treatment.

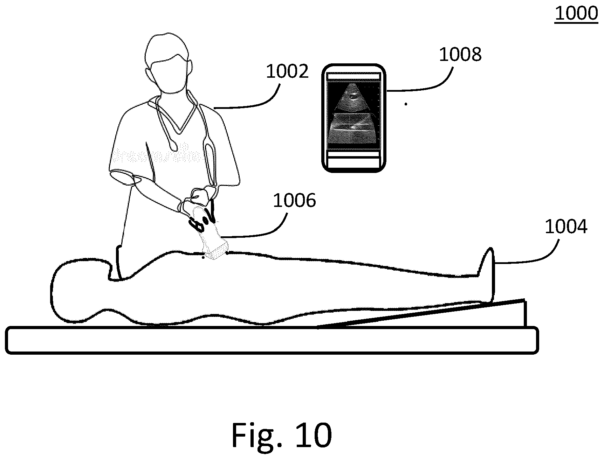

Figure 10 depicts pictorially one process 1000 where a clinician 1002 is performing an ultrasound procedure on a patient 1004 and to that end is positioning and orienting the probe 1006 while viewing an image generated by the probe 1006 and displayed on the handheld device 1008. In this operation the clinician 1002 may activate a preset on the user interface of the handheld system 1008 and that preset may activate the neural filter such that image generated by the probe 1006 is translated to have distributions matching those of images associated with cart-based systems. While moving the probe 1006 across the habitus of the patient 1004, the clinician may use the handheld ultrasound system with the neural filter active. Once the clinician 1002 has located the probe 1006 and oriented the probe 1006 as desired for the targeted anatomical view, the clinician 1002 may deactivate, by accessing a user interface button on the handheld device 1008, the neural filter so that ground images generated by the probe 1006 are displayed on the handheld device 1008. In this way, a clinician 1002 may employ the neural filter translation mechanism to use images that match the aesthetic, that is the look and feel, of cart based ultrasound systems while positioning the probe 1006 to collect a target anatomical view. Once the probe 1006 is positioned by the clinician 1002, the clinician 1002 may access a user interface switch to deactivate the neural network and see ground images generated by the probe 1006 as the clinician makes treatment and diagnostic determinations.

In several of the embodiments discussed above, the neural filter has been activated in concert with activation of a preset such as the cardiac preset. Although in some embodiments the neural filter may be activated independent of any preset, in other embodiments the neural filter is activated in response to selection of a preset. Although cardiac preset has been discussed, it will be understood by those of skill in the art that any useful preset may cause the activation of the neural filters disclosed herein. Table 1 presents a short list of presets of the type that may be used with the neural filters described herein. Table 1

**TABLE 1**

| Preset |
|---|
| Abdomen |
| Abdomen Deep |
| Aorta & Gallbladder |
| Bladder |
| Cardiac |
| Cardiac Deep |
| Coherence Imaging |
| FAST |
| Lung |
| MSK-Soft Tissue |
| Musculoskeletal |
| Nerve |
| OB 1/GYN |
| OB 2/3 |
| Ophthalmic |
| Pediatric Abdomen |
| Pediatric Cardiac |
| Pediatric Lung |
| Small Organ |
| Vascular: Access |
| Vascular: Carotid |
| Vascular: Deep Vein |

Each preset is a mode adapted for a particular type of imaging study. Presets may help with imaging studies and may be employed within systems that have the neural filter feature described herein, but optionally, presets may be employed within systems that do not have such a neural filter feature. In further optional embodiments, the clinician may be able to override a preset parameter that activates a neural filter to either activate or deactivate the neural filter for all operations regardless of the selected preset.

The systems and methods described herein reference circuits, CPUs and other devices, and those of skill in the art will understand that these embodiments are examples, and the actual implementation of these circuits may be carried out as software modules running on microprocessor devices and may comprise firmware, software, hardware, or any combination thereof that is configured to perform as the systems and processes described herein. Further, some embodiments may also be implemented by the preparation of application-specific integrated circuits or by interconnecting an appropriate network of conventional component circuits. To illustrate this interchangeability of hardware and software, various illustrative components, blocks, modules, circuits, and steps have been described herein generally in terms of their functionality. Whether such functionality is implemented as hardware or software depends upon the application and design constraints imposed on the overall system. Skilled artisans may implement the described functionality in varying ways for each particular application, but such implementation decisions should not be interpreted as causing a departure from the embodiments described herein.

Various aspects of the present disclosure may be used alone, in combination, or in a variety of arrangements not specifically described in the embodiments described in the foregoing and is therefore not limited in its application to the details and arrangement of components set forth in the foregoing description or illustrated in the drawings. For example, aspects described in one embodiment may be combined in any manner with aspects described in other embodiments.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

Use of ordinal terms such as "first," "second," "third," etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements.

Having described above several aspects of at least one embodiment, it is to be appreciated various alterations, modifications, and improvements will readily occur to those skilled in the art. Such alterations, modifications, and improvements are intended to be object of this disclosure. Accordingly, the foregoing description and drawings are by way of example only.

Accordingly, it will be understood that the invention is not to be limited to the embodiments disclosed herein and which include, but are not limited to:
A system for generating ultrasound images during a medical imaging procedure, comprising an ultrasound imaging device for generating a stream of image data,
an image processor for processing the stream of image data to generate images for use by a clinician,
a user interface for controlling operation of the image processor and having a neural filter UI switch that activates a neural filter, and
a neural filter responsive to the UI switch and for processing image data within the stream of images by adjusting an output distribution of the image data to conform with an output distribution of image data generated by cart-based ultrasound images and to maintain content of the image data within the generated stream of image data.

The system of above, wherein the UI switch is associated with a cardiac preset configuration for generating cardiac image data.

The system of above, wherein adjusting an output distribution includes adjusting an output distribution of an image to match an image output distribution associated with a cart-based ultrasound imaging device.

The system of above, wherein the neural filter includes a mapping function for translating image data generated from a handheld ultrasound device to images of the type generated by cart-based ultrasound devices.

The system of above, wherein the neural filter processes paired image data to generate the mapping function.

The system of above, wherein the neural filter processes unpaired image data to generate the mapping function.

The system of above having a training module that employs a cycle-consistent adversarial network to process unpaired image data to generate the mapping function.

## Claims

1. A system of generating ultrasound images during a medical imaging procedure comprising,
a handheld ultrasound imaging device for generating a stream of image data from the habitus of the patient,
an image processor for processing the stream of image data to produce images,
a neural filter which receives the stream of image data from the handheld ultrasound imaging device and processes it to generate a new stream of image data that produces images such that the output distribution of images conforms to the visual properties of the image distribution of ultrasound images of the type produced by cart-based ultrasound systems, and a user interface control for controlling operation of the neural filter and having a UI switch for activating the neural filter.

2. The system of Claim 1 wherein the UI switch comprises a preset configuration for configuring the handheld ultrasound imaging device to generate image data for an associated image study requirement associated with the preset and for processing generated image data with the neural filter to conform the visual properties of the generated image data to have an image distribution of ultrasound images of the type produced by cart-based ultrasound systems for the respective preset image study requirements .

3. The system of Claim 1 wherein adjusting the output distribution to conform to the visual properties of the image distribution of the type produced by cart-based ultrasound systems includes adjusting the image data such that the measures of the visual properties of sharpness, resolution, and noise of the resulting output image conform to the measures of the visual properties of sharpness, resolution and noise of the output distribution of ultrasound images produced by cart-based ultrasound imaging systems.

4. The system of Claim 1 wherein the neural filter includes a mapping function for translating image data produced by a handheld ultrasound imaging system into image data of the type produced by cart-based ultrasound imaging systems by employing a training module to define for the neural filter visual properties of image data produced by handheld ultrasound imaging devices and cart-based ultrasound imaging systems respectively.

5. The system of Claim 4 wherein the training module processes the image data of paired images across an image distribution of the type produced by handheld ultrasound imaging systems and an image distribution of the type produced by cart-based ultrasound imaging systems to generate the mapping function.

6. The system of Claim 4 wherein the training module processes the image data of unpaired images across an image distribution of the type produced by handheld ultrasound imaging systems and an image distribution of the type produced by cart-based ultrasound imaging systems to generate the mapping function.

7. The system of Claim 4 wherein the training module employs a cycle-consistent adversarial network to evaluate unpaired images translated from a first image distribution into a second image distribution to determine the accuracy of the translation, and evaluating images translated from a first image distribution into a second image distribution and then back into the first image distribution to determine the content lost across the translation to generate the mapping function.

8. A method of generating ultrasound images during a medical imaging procedure comprising, generating with a handheld ultrasound imaging device a stream of image data from the habitus of the patient,
processing the stream of image data to produce images,
receiving the stream of image data and processing it to generate a new stream of image data that produces images such that the output distribution of images conforms to the visual properties of the image distribution of ultrasound images of the type produced by cart-based ultrasound systems, and
controlling operation of the neural filter comprising a UI switch for activating the neural filter.

9. The method of Claim 8 wherein controlling operation of the neural filter includes accessing a preset configuration for configuring the handheld ultrasound imaging device to generate image data for image study requirements associated with the preset and processing generated image data with the neural filter to conform to the visual properties of the generated image data to have an image distribution of ultrasound images of the type produced by cart-based ultrasound systems for the respective preset image study requirements .

10. The method of Claim 8 wherein adjusting the output distribution to conform to the visual properties of the image distribution of the type produced by cart-based ultrasound systems includes adjusting the image data such that the measures of the visual properties of sharpness, resolution, and noise of the resulting output image conforms to the measures of the visual properties sharpness, resolution and noise of the output distribution of ultrasound images produced by cart-based ultrasound imaging systems.

11. The method of Claim 8 wherein receiving the stream of image data and processing it to generate a new stream of image data includes employing a neural filter which includes employing a mapping function for translating image data produced by a handheld ultrasound imaging system into image data of the type produced by cart-based ultrasound imaging systems by employing a training module to define for the neural filter visual properties of image data produced by handheld ultrasound imaging devices and cart-based ultrasound imaging systems respectively.

12. The method of Claim 11 wherein employing a training module includes processing the image data of paired images across an image distribution of the type produced by handheld ultrasound imaging systems and an image distribution of the type produced by cart-based ultrasound imaging systems to generate the mapping function.

13. The method of Claim 11 wherein employing a training module includes processing the image data of unpaired images across an image distribution of the type produced by handheld ultrasound imaging systems and an image distribution of the type produced by cart-based ultrasound imaging systems to generate the mapping function.

14. The method of Claim 11 wherein employing a training module includes employing a cycle-consistent adversarial network to evaluate unpaired images translated from a first image distribution into a second image distribution to determine the accuracy of the translation, and evaluating images translated from a first image distribution into a second image distribution and then back into the first image distribution to determine the content lost across the translation to generate the mapping function.
